# EUROPÄISCHE PATENTSCHRIFT

(11) **EP 0 770 375 B1**
(45) Veröffentlichungstag und Bekanntmachung des Hinweises auf die Patenterteilung: **19.11.1997**
(21) Anmeldenummer: 96114682.6
(22) Anmeldetag: 13.09.1996
(51) Int. Cl.: A61K 7/13

(54) **Haarfärbemittel**
Hair dyeing composition
Composition pour la teinture des cheveux

(30) Priorität: 21.10.1995 DE 19539264
(43) Veröffentlichungstag der Anmeldung: 02.05.1997
(73) Patentinhaber: GOLDWELL GmbH, D-64297 Darmstadt (DE)
(72) Erfinder: Lorenz, Heribert, 64401 Gross-Bieberau (DE); Golinski, Frank, Dr., 64297 Darmstadt (DE); Hiermer, Christine, 64285 Darmstadt (DE)

(56) Entgegenhaltungen:
- EP-A- 0 467 026
- EP-A- 0 657 159
- DE-A- 4 400 757

## Beschreibung

Die vorliegende Erfindung betrifft ein Haarfärbemittel auf Basis eines mit Peroxid reagierenden Entwickler-Kuppler-Systems, daß einen ausdrucksvollen, brillanten und dauerhaften rotvioletten Grundton liefert, der entweder als solcher angewandt werden, oder, in Kombination mit weiteren Entwickler- und/oder Kupplersubstanzen, zur Erzielung weiterer Farbnuancen benutzt werden kann.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen ist insofern nicht völlig problemfrei, als sie bei extrem empfindlichen Personen in speziellen Fällen zu Hautsensibilisierungen führen können (bei sogenannten "Para-Allergikern").

Es wurde bereits versucht, dieses Problem durch Verwendung alternativer Entwicklersubstanzen zu lösen. Dies ist in beschränktem Umfang möglich durch den Einsatz von Tetraaminopyrimidin oder 2-(2,5-Diaminophenyl)ethanol (vgl. EP-A 7537 und EP-B 400 330); jedoch müssen dann erhebliche Abstriche in der Färbeintensität und den Variationsmöglichkeiten der verschiedenen Farbtöne hingenommen werden.

Eine weitgehend optimale Lösung des Problems, nämlich die Abwesenheit von Hautsensibilisierungen einerseits und eine große Variationsbreite der Erzielung möglicher Farbnuancen andererseits, wird durch den in der EP-B 467 026 beschriebenen Einsatz von Hydroxytriaminopyrimidinen als Entwicklersubstanzen in Haarfärbemitteln erreicht.

Dies läßt sich durch Kombination mit verschiedenen speziellen Kupplersubstanzen, wie sie beispielsweise in der DE-A 41 15 148, der EP-A 542 129, der DE-A 44 00 757, der EP-A 657 158 oder der DE-A 42 19 981 beschrieben sind, noch verbessern.

Selbst dadurch bleiben jedoch noch farbtechnische Wünsche offen.

Die Erfindung geht daher von der Aufgabenstellung aus, ein Haarfärbemittel zu schaffen, das ein Hydroxytriaminopyrimidin bzw. dessen wasserlösliche Salze als Entwicklersubstanz enthält und zur Herstellung eines rotvioletten Farbtons geeignet ist.

Diese Aufgabe wird dadurch gelöst, daß ein solches Haarfärbemittel ein mit Peroxid reagierendes Entwickler-Kuppler-System enthält, das aus einer Kombination aus mindestens einem Hydroxytriaminopyrimidin bzw. dessen wasserlöslichen Salzen, 5-Amino-2-methylphenol und 4-Amino-3-methylphenol besteht.

Bei Anwendung dieser Zusammensetzung auf Basis einer üblichen Grundlage wird nach der Oxidation mit Peroxid eine sehr ausdrucksvolle, intensive rotviolette Grundfärbung erhalten, die durch Zusatz entsprechender weiterer Kupplersubstanzen zu anderen Farbnuancen variiert werden kann.

Solche bevorzugten weiteren Kupplersubstanzen sind insbesondere Resorcin, 2-Methylresorcin, 4-Chlorresorcin, 3-Aminophenol, 2-Aminophenol, 2-Amino-4-β-hydroxyethylaminoanisol bzw. dessen wasserlösliche Salze und α-Naphthol.

Damit soll jedoch der Zusatz weiterer Kupplersubstanzen keineswegs ausgeschlossen sein.

Auch die Mitverwendung weiterer, an sich bekannter Entwicklersubstanzen ist möglich. Neben den bereits eingangs genannten sind hierbei insbesondere noch 4-Aminophenol und/oder 5-Aminosalicylsäure zu erwähnen.

Als Hydroxytriaminopyrimidine werden insbesondere das aus der bereits erwähnten EP-B 467 026 bekannte 2,4,5-Triamino-6-hydroxypyrimidin und das 4,5,6-Triamino-2-hydroxypyrimidin eingesetzt, insbesondere auch als Sulfat-Salze.

Die Konzentration der Entwicklersubstanzen liegt zwischen etwa 0,05 und 5, vorzugsweise 0,1 und 4, insbesondere 0,25 bis 0,5 und 2,5 bis 3 Gew.-% der Gesamtzusammensetzung des Haarfärbemittels (ohne Oxidationsmittel), wobei sich die Angaben jeweils auf den Anteil an freier Base beziehen.

Die Kupplersubstanzen als Reaktionspartner der Entwicklersubstanz(en) liegen in den erfindungsgemäßen Haarfärbemitteln etwa im gleichen Anteil wie die Entwicklersubstanzen vor, d.h., also in Mengen von 0,05 bis 5,0 vorzugsweise 0,1 bis 4, insbesondere 0,5 bis 3 Gew.-% der Gesamtzusammensetzung (ohne Oxidationsmittel), wobei sich die Angaben jeweils auf den Anteil an freier Base beziehen.

Auch hier ist es, wie bereits erwähnt, möglich und zuweilen auch zweckmäßig, weitere bekannte Kupplersubstanzen mitzuverwenden, falls dies zur Erzielung bestimmter Farbnuancen erwünscht und erforderlich ist.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.
Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5, insbesondere 0,1 bis 1 Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Auflage (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d.h. nach Vermischung mit Peroxid, kann sowohl im schwach sauren, d.h. einem pH-Bereich von 5,5 bis 6,9, im neutralen als auch im alkalischen Bereich, d.h. zwischen pH 7,1 und 9,5, liegen.

Im folgenden werden verschiedene Ausführungsbeispiele zur Erläuterung der Erfindung gegeben.

### Grundlage

| | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%-ig | 8,5 |
| Ammoniumchlorid | 0,5 |
| Panthenol | 0,8 |
| Wasser | ad 100,0 |

Die erfindungsgemäße Entwickler-Kuppler-Kombination wurde jeweils, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%-iger Wasserstoffperoxid-Lösung und zwanzigminütiger Einwirkung bei Zimmertemperatur, folgendem Auswaschen und Trocknen.

Es wurden die folgenden Färbungen erzielt:

| | | |
|---|---|---|
| **Beispiel 1**: | 0,24 (Gew.-%) | 4-Hydroxy-2,5,6-triaminopyrimidinsulfat |
| | 0,25 | 4-Amino-3-methylphenol |
| | 0,18 | 5-Amino-2-methylphenol |
| Färbung: | Kräftiges Rotviolett. | |
| **Beispiel 1a:** | Weglassen von 4-Amino-3-methylphenol führte zu einer blassen, rötlichen Färbung. | |
| **Beispiel 2:** | 0,27 (Gew.-%) | 4-Hydroxy-2,5,6-triaminopyrimidinsulfat |
| | 0,14 | 4-Amino-3-methylphenol |
| | 0,14 | 5-Amino-2-methylphenol |
| | 0,12 | 3-Aminophenol |
| Färbung: | Dunkles Rotviolett. | |
| **Beispiel 3:** | 0,27 (Gew.-%) | 4-Hydroxy-2,5,6-triaminopyrimidinsulfat |
| | 0,14 | 4-Amino-3-methylphenol |
| | 0,14 | 5-Amino-2-methylphenol |
| | 0,16 | α-Naphthol |
| Färbung: | Intensives Rotviolett mit aparter Blautönung. | |
| **Beispiel 4:** | 0,27 (Gew.-%) | 4-Hydroxy-2,5,6-triaminopyrimidinsulfat |
| | 0,14 | 4-Amino-3-methylphenol |
| | 0,14 | 5-Amino-2-methylphenol |
| | 0,33 | 2-Amino-4-(β-hydroxyethylamino)anisolsulfat |
| Färbung: | Braunviolett. | |

## Patentansprüche

1. Haarfärbemittel auf Basis eines mit Peroxid reagierenden Entwickler-Kuppler-Systems, dadurch gekennzeichnet, daß es eine Kombination aus
a) mindestens einem Triaminohydroxypyrimidin bzw. dessen wasserlöslichen Salzen;
b) 5-Amino-2-methylphenol; und
c) 4-Amino-3-methylphenol
enthält.

2. Haarfärbemittel nach Anspruch 1 dadurch gekennzeichnet, daß es mindestens eine zusätzliche Kupplersubstanz, ausgewählt aus der Gruppe Resorcin, 2-Methylresorcin, 4-Chlorresorcin, 3-Aminophenol, α-Naphthol und/oder 2-Amino-4-(β-hydroxyethylamino)anisol bzw. dessen wasserlöslichen Salzen, enthält.

## Claims

1. Hair dyeing composition containing a developing/coupling system reacting with peroxides, comprising
a) at least one triaminohydroxypyrimidine or the water-soluble salts thereof;
b) 5-amino-2-methyl phenol; and
c) 4-amino-3-methyl phenol.

2. Hair dyeing composition according to claim 1, comprising at least one additional coupling agent, selected from the group of resorcinol, 2-methyl resorcinol, 4chlororesorcinol, 3-aminophenol, α-naphthol and (or) 2-amino-4-(β-hydroxyethyl amino)anisole or the water-soluble salts thereof.

## Revendications

1. Composition pour la teinture des cheveux, contenant un système des substances révélatrices et des substances de couplage réagissant avec des peroxydes, comprenant
a) au moins d'une triaminohydroxypyrimidine ou ses sels hydrosolubles;
b) 5-amino-2-méthylphénol; et
c) 4-amino-3-méthylphénol.

2. Composition pour la teinture des cheveux selon la revendication 1, comprenant au moins d'une substance de couplage sélectionné parmi résorcine, 2-méthylrésorcine, 4-chlororésorcine, 3-aminophénol, α-naphthol et (ou) 2-amino-4-(β-hydroxyéthylamino) anisole ou ses sels hydrosolubles.
